# EUROPEAN PATENT APPLICATION

(11) **EP 4 786 507 A1**
(43) Date of publication of application: **05.08.2026**
(21) Application number: 24872243.1
(22) Date of filing: 25.09.2024
(51) Int. Cl.: C08F 220/36, C12N 15/10

(54) **STABILIZER FOR NUCLEIC ACID SOLUTION**

(30) Priority: 27.09.2023 JP 2023166492
(71) Applicant: NOF Corporation, Shibuya-ku Tokyo 150-6019 (JP)
(72) Inventor: KOHATSU, Haruki, Kawasaki-shi, Kanagawa 210-0865 (JP); SUZUKI, Hirotaka, Kawasaki-shi, Kanagawa 210-0865 (JP); MATSUDA, Masaru, Kawasaki-shi, Kanagawa 210-0865 (JP)
(74) Representative: Vossius & Partner Patentanwälte Rechtsanwälte mbB
(86) International application number: PCT/JP2024/034087
(87) International publication number: WO 2025/070457

(57) **Abstract**

The present invention provides a stabilizer for a nucleic acid solution, which is a polymer containing a constitutional unit derived from a monomer represented by the following formula (1) (the symbols in the following formula are as defined in the specification).

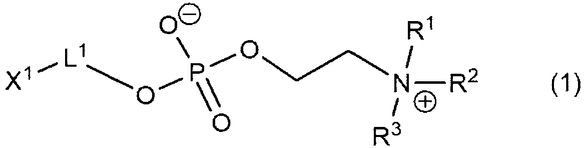

## Description

### [Technical Field]

The present invention relates to a stabilizer for nucleic acid solutions and a method for improving the storage stability of nucleic acids in nucleic acid solutions by using the stabilizer.

### [Background Art]

RNA plays a role in biosynthesizing proteins based on the genetic information stored in DNA. In recent years, various industrial products, such as useful protein, pharmaceutical product, and the like, have been developed using this RNA.

Storage of this RNA is difficult because it is easily degraded in solutions and unstable. Therefore, extracted RNA needs to be immediately stored at low temperatures. For example, a method of freeze-drying and storing RNA to improve stability thereof (e.g., Patent Literature 1) and a method of storing RNA at -20°C without freezing by adding a 50% ethanol aqueous solution to a Tris-EDTA (TE) buffer have been conventionally used.

### [Citation List]

### [Patent Literature]

[Patent Literature 1]
JP 2021-119161 A

### [Summary of Invention]

### [Technical Problem]

The storage stability of RNA can be improved by freeze-drying a sample containing RNA or by adding a buffer to the aforementioned sample and storing same at a low temperature without freezing. However, the RNA can be degraded by freezing operation of the RNA during freeze-drying or by thawing operation of the frozen sample before use. In addition, adding a buffer to a sample containing RNA does not sufficiently improve the storage stability of the RNA.

The present invention has been made in view of the above-mentioned problems, and aims to provide a stabilizer that can improve the storage stability of a nucleic acid by simply adding the stabilizer to a solution of the nucleic acid (particularly RNA solution).

### [Solution to Problem]

The present inventors have conducted intensive studies and found that the storage stability of a nucleic acid can be improved by adding a polymer containing a constitutional unit derived from a monomer represented by the following formula (1): 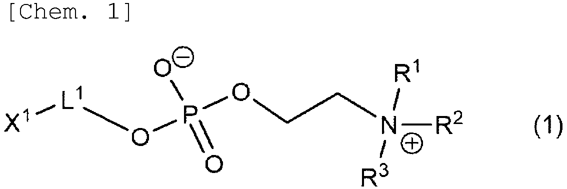 to a solution of the nucleic acid. Based on this finding, the present invention provides the following.
[1] A stabilizer for a nucleic acid solution, which is a polymer comprising a constitutional unit derived from a monomer represented by the formula (1): 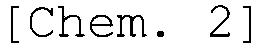 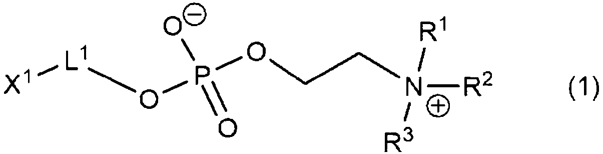 wherein
   X¹ is a (meth)acryloyloxy group or a (meth)acryloylamino group,
   L¹ is an alkylene group having 2 to 4 carbon atoms and optionally having one hydroxy group, or an alkyleneoxyalkylene group having 2 to 4 carbon atoms, and
   R¹ to R³ are each independently an alkyl group having 1 to 3 carbon atoms.
[2] The stabilizer of the aforementioned [1], wherein the aforementioned polymer is a copolymer further comprising a constitutional unit derived from a monomer represented by the formula (2): 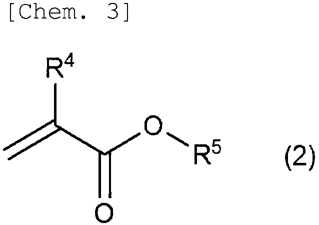 wherein
   R⁴ is a hydrogen atom or a methyl group, and
   R⁵ is is a hydrogen atom, an alkyl group having 1 to 20 carbon atoms, or *-(OCH₂CH₂)ₙOR⁶ group (in the aforementioned formula, * is a binding position, n is a number from 1 to 10, and R⁶ is a hydrogen atom, a methyl group, or an ethyl group.
[3] The stabilizer of the aforementioned [1] or [2], wherein the aforementioned polymer is a copolymer further comprising a constitutional unit derived from a monomer represented by the formula (3): 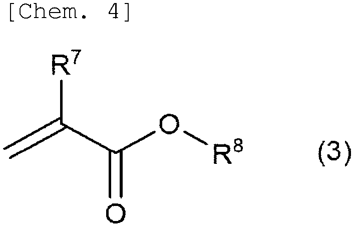 wherein
   R⁷ is a hydrogen atom or a methyl group, and
   R⁸ is an alkyl group having 3 to 6 carbon atoms, and two or more hydroxy groups.
[4] The stabilizer of any one of the aforementioned [1] to [3], wherein the aforementioned nucleic acid solution is an RNA solution.
[5] A method for improving the storage stability of a nucleic acid, comprising adding the stabilizer of any one of the aforementioned [1] to [4] to a solution of the nucleic acid at a concentration of 0.0001 to 10 w/v%.

### [Advantageous Effects of Invention]

The stabilizer of the present invention can improve the storage stability of a nucleic acid by simply adding the stabilizer to a solution of the nucleic acid (particularly RNA solution).

### [Description of Embodiments]

The present invention is described in detail below.

The descriptions in the present specification can be combined with each other unless it is clear that they cannot be combined. When stepwise numerical ranges are described in the present specification, the lower limit and the upper limit of each numerical range can be combined. In the case of, for example, "preferably 10 to 100, more preferably 20 to 90", "the preferred lower limit 10" can be combined with "the more preferred upper limit 90" (i.e., the numerical range of "10 to 90" is also within the range of the present specification).

### [Stabilizer for nucleic acid solution]

The present invention provides a stabilizer for a nucleic acid solution (hereinafter sometimes abbreviated as "the stabilizer of the present invention"), which is a polymer containing a constitutional unit derived from a monomer represented by the formula (1) (hereinafter sometimes abbreviated as "the polymer of the present invention").

In the present specification, the "nucleic acid" means "RNA or DNA".

In the present specification, "the stabilizer for nucleic acid solution" means an additive used in a nucleic acid solution to improve the storage stability of the nucleic acid. The nucleic acid solution is preferably an RNA solution.

The stabilizer of the present invention is a polymer containing a constitutional unit derived from a monomer represented by the formula (1): 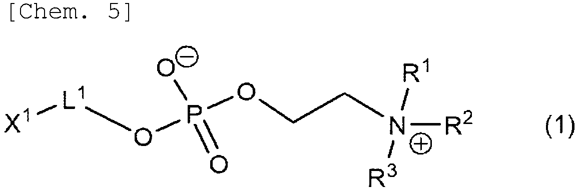 wherein
X¹ is a (meth)acryloyloxy group or a (meth)acryloylamino group,
L¹ is an alkylene group having 2 to 4 carbon atoms and optionally having one hydroxy group, or an alkyleneoxyalkylene group having 2 to 4 carbon atoms, and
R¹ to R³ are each independently an alkyl group having 1 to 3 carbon atoms. In other words, the present invention provides use of a polymer containing a constitutional unit derived from a monomer represented by the formula (1) as a stabilizer for a nucleic acid solution.

Only one kind of the polymer of the present invention may be used, or two or more kinds thereof may be used in combination. In the following, the "monomer represented by the formula (1)" is sometimes abbreviated as "monomer (1)". The "monomer represented by other formula" is also sometimes similarly abbreviated as "a monomer represented by the formula (1)". The "constitutional unit derived from monomer (1)" is sometimes abbreviated as "constitutional unit (1)". The "constitutional unit derived from other monomer" is also sometimes similarly abbreviated as "a constitutional unit derived from monomer (1)". The constitutional unit derived from monomer (1) means a constitutional unit having a structure formed by the reaction of a carbon-carbon double bond of the (meth)acryloyl group contained in monomer (1). The constitutional unit derived from other monomers means the same as the constitutional unit derived from monomer (1).

Only one kind of monomer (1) may be used, or two or more kinds thereof may be used in combination. That is, the polymer of the present invention may be a homopolymer consisting of one kind of constitutional unit (1), or a copolymer containing two or more kinds of constitutional units (1). The aforementioned copolymer may be a random copolymer, a block copolymer, or a copolymer containing both a random portion and a block portion.

The groups in the formula (1) are described in order below. In the formula (1), X¹ is a (meth)acryloyloxy group (i.e., CH₂=CR-CO-O-, R: hydrogen atom or a methyl group) or a (meth)acryloylamino group (i.e., CH₂=CR-CO-NH-, R: hydrogen atom or a methyl group). From the aspect of the availability of the starting materials, X¹ is preferably a (meth)acryloyloxy group, more preferably a methacryloyloxy group.

In the formula (1), L¹ is an alkylene group having 2 to 4 carbon atoms and optionally having one hydroxy group, or an alkyleneoxyalkylene group having 2 to 4 carbon atoms. The aforementioned alkylene group may be linear or branched chain. Examples of the alkylene group having 2 to 4 carbon atoms and optionally having one hydroxy group include -C₂H₄-. Examples of the alkyleneoxyalkylene group having 2 to 4 carbon atoms include -C₂H₄-O-C₂H₄-. From the aspect of the availability of the starting materials, L¹ is preferably -C₂H₄- or -C₂H₄-O-C₂H₄-, more preferably -C₂H₄- (i.e., ethylene group).

In the formula (1), R¹ to R³ are each independently an alkyl group having 1 to 3 carbon atoms. The aforementioned alkyl group may be linear or branched chain. Examples of the alkyl group having 1 to 3 carbon atoms include methyl group, ethyl group, propyl group, and the like. From the aspect of the availability of the starting materials, R¹ to R³ are preferably methyl groups.

Preferred monomer (1) is a monomer in which X¹ is a (meth)acryloyloxy group, L¹ is -C₂H₄- or -C₂H₄-O-C₂H₄-, and R¹ to R³ are methyl groups. More preferred monomer (1) is a monomer in which X¹ is a (meth)acryloyloxy group, L¹ is an ethylene group, and R¹ to R³ are methyl groups (i.e., 2-(meth)acryloyloxyethyl phosphorylcholine). Further preferred monomer (1) is 2-methacryloyloxyethyl phosphorylcholine. A commercially available product can be used for monomer (1).

In the present specification, the "2-(meth)acryloyloxyethyl phosphorylcholine" basically means "2-acryloyloxyethyl phosphorylcholine or 2-methacryloyloxyethyl phosphorylcholine". When a plurality of 2-(meth)acryloyloxyethyl phosphorylcholine may be present, the "2-(meth)acryloyloxyethyl phosphorylcholine" means "2-acryloyloxyethyl phosphorylcholine and/or 2-methacryloyloxyethyl phosphorylcholine". Other terms similar to the "2-(meth)acryloyloxyethyl phosphorylcholine" also mean the same as the"(2-(meth)acryloyloxyethyl phosphorylcholine".

One embodiment of the polymer of the present invention is a polymer consisting of constitutional unit (1) (hereinafter sometimes abbreviated as "polymer (1)"). In the present specification, the "polymer consisting of constitutional unit (1)" refers to a polymer in which all constitutional units (repeating units) in the polymer chain consist of constitutional unit (1). Expressions similar to the "polymer consisting of constitutional unit (1)" mean the same as the "polymer consisting of constitutional unit (1)". The aforementioned "constitutional unit" means "a constitutional unit derived from a monomer and does no include a structure derived from a monomer (for example, structure derived from polymerization initiator).

Polymer (1) may be a homopolymer consisting of one kind of constitutional unit (1) or a copolymer consisting of two or more kinds of constitutional units (1). It is preferably copolymer consisting of two or more kinds of constitutional units (1).

While the weight average molecular weight of polymer (1) is not particularly limited, it is preferably 20,000 to 2,000,000, more preferably 100,000 to 1,500,000, further preferably 500,000 to 1,500,000. The weight average molecular weight can be determined based on polyethylene glycol by gel permeation chromatography, for example, using EcoSEC system (manufactured by Tosoh Corporation) and the like.

The polymer of the present invention may further contain, in addition to constitutional unit (1), a constitutional unit derived from a monomer represented by the formula (2): 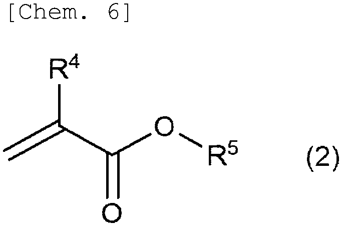 wherein
R⁴ is a hydrogen atom or a methyl group, and
R⁵ is a hydrogen atom, an alkyl group having 1 to 20 carbon atoms, or *-(OCH₂CH₂)ₙOR⁶ group (in the aforementioned formula, * is a binding position, n is a number from 1 to 10, and R⁶ is a hydrogen atom, a methyl group or an ethyl group).

The groups in the formula (2) are described in order below. In the formula (2), R⁴ is a hydrogen atom or a methyl group. From the aspect of the storage stability of the polymer, R⁴ is preferably a methyl group.

In the formula (2), R⁵ is a hydrogen atom, an alkyl group having 1 to 20 carbon atoms, or *-(OCH₂CH₂)ₙOR⁶ group.

A monomer in which R⁵ in the formula (2) is a hydrogen atom or an alkyl group having 1 to 20 carbon atoms (hereinafter sometimes to be abbreviated as "monomer (2a)") is explained. The aforementioned alkyl group may be linear or branched chain. In monomer (2a), R⁵ is preferably a hydrogen atom or an alkyl group having 10 to 20 carbon atoms, more preferably a hydrogen atom or an alkyl group having 15 to 19 carbon atoms, further preferably a hydrogen atom or a linear alkyl group having 17 to 19 carbon atoms. When the polymer of the present invention further contains a constitutional unit derived from a monomer represented by the below-mentioned formula (3), R⁵ is preferably an alkyl group having 3 to 6 carbon atoms.

Specific examples of monomer (2a) include (meth)acrylic acid, methyl (meth)acrylate, ethyl (meth)acrylate, propyl (meth)acrylate, butyl (meth)acrylate, pentyl (meth)acrylate, hexyl (meth)acrylate, heptyl (meth)acrylate, octyl (meth)acrylate, nonyl (meth)acrylate, decyl (meth)acrylate, undecyl (meth)acrylate, dodecyl (meth)acrylate, tridecyl (meth)acrylate, tetradecyl (meth)acrylate, pentadecyl (meth)acrylate, cetyl (meth)acrylate, heptadecyl (meth)acrylate, stearyl (meth)acrylate, and the like. Only one kind of monomer (2a) may be used, or two or more kinds thereof may be used in combination. A commercially available product can be used for monomer (2a).

In the aforementioned specific examples of monomer (2a),
(i) (meth)acrylic acid, ethyl (meth)acrylate, propyl (meth)acrylate, butyl (meth)acrylate, pentyl (meth)acrylate, hexyl (meth)acrylate, decyl (meth)acrylate, undecyl (meth)acrylate, dodecyl (meth)acrylate, tridecyl (meth)acrylate, tetradecyl (meth)acrylate, pentadecyl (meth)acrylate, cetyl (meth)acrylate, heptadecyl (meth)acrylate, and stearyl (meth)acrylate are preferred,
(ii) (meth)acrylic acid, propyl (meth)acrylate, butyl (meth)acrylate, pentyl (meth)acrylate, pentadecyl (meth)acrylate, cetyl (meth)acrylate, heptadecyl (meth)acrylate, and stearyl (meth)acrylate are more preferred,
(iii) (meth)acrylic acid, butyl (meth)acrylate and stearyl (meth)acrylate are further preferred, and
(iv) methacrylic acid, butyl methacrylate and stearyl methacrylate are particularly preferred.

One embodiment of the polymer of the present invention is a copolymer consisting of constitutional unit (1) and constitutional unit (2a) (hereinafter sometimes abbreviated as "copolymer (1-2a)"). Only one kind each of monomer (1) and monomer (2a) may be used, or two or more kinds thereof may be used in combination. That is, copolymer (1-2a) may be a copolymer containing one or more kinds of constitutional units (1) and one or more kinds of constitutional units (2a). The aforementioned copolymer may be a random copolymer, a block copolymer, or a copolymer containing both a random portion and a block portion.

In copolymer (1-2a), the quantitative ratio of constitutional unit (1) (i.e., quantitative ratio of monomer (1) used in polymerization) is preferably 5 to 95 mol, more preferably 10 to 95 mol, further preferably 15 to 95 mol, particularly preferably 20 to 90 mol, and the quantitative ratio of constitutional unit (2a) (i.e., quantitative ratio of monomer (2a) used in polymerization) is preferably 5 to 95 mol, more preferably 5 to 90 mol, further preferably 5 to 85 mol, particularly preferably 10 to 80 mol, each with respect to the total 100 mol of the constitutional unit (1) and constitutional unit (2a) (i.e., total 100 mol of monomer (1) and monomer (2a) used in polymerization).

While the weight average molecular weight of copolymer (1-2a) is not particularly limited, it is preferably 10,000 to 2,000,000, more preferably 20,000 to 1,500,000, further preferably 30,000 to 1,000,000. The weight average molecular weight can be determined based on polyethylene glycol by gel permeation chromatography, for example, using EcoSEC system (manufactured by Tosoh Corporation) and the like.

A monomer in which R⁵ in the formula (2) is a -(OCH₂CH₂)ₙOR⁶ group (wherein * is a binding position, n is a number from 1 to 10, and R⁶ is a hydrogen atom, a methyl group, or an ethyl group) (hereinafter sometimes to be abbreviated as "monomer (2b)") is explained.

R⁶ is preferably a methyl group or an ethyl group, more preferably a methyl group.

In the *-(OCH₂CH₂)ₙOR⁶ group, n is a number from 1 to 10, preferably a number from 5 to 10, more preferably a number from 8 to 10.

Specific examples of monomer (2b) include polyethylene glycol mono(meth)acrylate, methoxypolyethylene glycol (meth)acrylate, ethoxypolyethylene glycol (meth)acrylate, and the like. Only one kind of monomer (2b) may be used, or two or more kinds thereof may be used in combination. From the viewpoint of the storage stability of polymers, methoxypolyethylene glycol (meth)acrylate, and ethoxypolyethylene glycol (meth)acrylate are preferred, and methoxypolyethylene glycol methacrylate is more preferred. A commercially available product can be used for monomer (2b).

One embodiment of the polymer of the present invention is a copolymer consisting of constitutional unit (1) and constitutional unit (2b) (hereinafter sometimes abbreviated as "copolymer (1-2b)"). Only one kind each of monomer (1) and monomer (2b) may be used, or two or more kinds thereof may be used in combination. That is, copolymer (1-2b) may be a copolymer containing one or more kinds of constitutional units (1) and one or more kinds of constitutional units (2b). The aforementioned copolymer may be a random copolymer, a block copolymer, or a copolymer containing both a random portion and a block portion.

In copolymer (1-2b), the quantitative ratio of constitutional unit (1) (i.e., quantitative ratio of monomer (1) used in polymerization) is preferably 40 to 95 mol, more preferably 60 to 95 mol, further preferably 80 to 95 mol, and the quantitative ratio of constitutional unit (2b) (i.e., quantitative ratio of monomer (2b) used in polymerization) is preferably 5 to 60 mol, more preferably 5 to 40 mol, further preferably 5 to 20 mol, each with respect to the total 100 mol of the constitutional unit (1) and constitutional unit (2b) (i.e., total 100 mol of monomer (1) and monomer (2b) used in polymerization).

While the weight average molecular weight of polymer (1-2b) is not particularly limited, it is preferably 50,000 to 1,000,000, more preferably 100,000 to 500,000, further preferably 100,000 to 300,000.

The polymer of the present invention may further contain, in addition to constitutional unit (1), a constitutional unit (hereinafter sometimes abbreviated as "constitutional unit (3)") derived from a monomer (hereinafter sometimes abbreviated as "monomer (3)") represented by the formula (3): 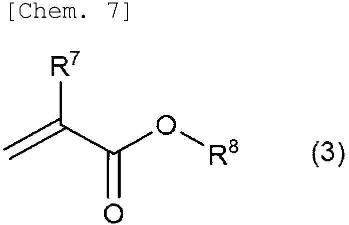 wherein R⁷ is a hydrogen atom or a methyl group, and R⁸ is an alkyl group having 3 to 6 carbon atoms and two or more hydroxy groups.

The groups in the formula (3) are described in order below. In the formula (3), R⁷ is a hydrogen atom or a methyl group. From the aspect of the storage stability of the polymer, R⁷ is preferably a methyl group.

In the formula (3), R⁸ is an alkyl group having 3 to 6 carbon atoms, and two or more hydroxy groups. The aforementioned alkyl group may be linear or branched chain. Examples of the alkyl group having 3 to 6 carbon atoms include propyl group, butyl group, pentyl group, hexyl group, and the like.

Specific examples of monomer (3) include, glycerol mono(meth)acrylate, threitol mono(meth)acrylate, erythritol mono(meth)acrylate, xylitol mono(meth)acrylate, arabitol mono(meth)acrylate, mannitol mono(meth)acrylate, galactitol mono(meth)acrylate, sorbitol mono(meth)acrylate, and the like. Among these, glycerol mono(meth)acrylate and xylitol mono(meth)acrylate are preferred, glycerol mono(meth)acrylate is more preferred, and glycerol monomethacrylate is further preferred. Only one kind of monomer (3) may be used, or two or more kinds thereof may be used in combination.

A commercially available product can be used for monomer (3), or may be produced by a known method. For example, monomer (3) can be produced by an esterification reaction of (meth)acrylic acid or a derivative thereof (e.g., acid chloride) with a polyhydric alcohol having 3 or more hydroxy groups. Esterification reaction is well known, those of ordinary skill in the art can set the conditions appropriately and perform the reaction.

One embodiment of the polymer of the present invention is a copolymer consisting of constitutional unit (1), constitutional unit (2), and constitutional unit (3) (hereinafter sometimes abbreviated as "copolymer (1-2-3)"). Only one kind each of the monomer (1), monomer (2), and monomer (3) may be used, or two or more kinds thereof may be used in combination. That is, copolymer (1-2-3) may be a copolymer containing one or more kinds of constitutional units (1), one or more kinds of constitutional units (2), and one or more kinds of constitutional units (3). The aforementioned copolymer may be a random copolymer, a block copolymer, or a copolymer containing both a random portion and a block portion.

Among copolymers (1-2-3), a copolymer in which the unit (2) is a constitutional unit derived from monomer (2a) (hereinafter sometimes abbreviated as "copolymer (1-2a-3)") is preferred. R⁵ in copolymer (1-2a-3) is preferably an alkyl group having 3 to 6 carbon atoms.

In copolymer (1-2-3), the amount ratio of constitutional unit (1) (i.e., the amount ratio of monomer (1) used in the polymerization) is preferably 30 to 80 mol, more preferably 30 to 70 mol, further preferably 30 to 60 mol, the amount ratio of constitutional unit (2) (i.e., the amount ratio of monomer (2a) used in the polymerization) is preferably 10 to 60 mol, more preferably 20 to 60 mol, further preferably 30 to 60 mol, and the amount ratio of constitutional unit (3) (i.e., the amount ratio of monomer (3) used in the polymerization) is preferably 10 to 60 mol, more preferably 10 to 50 mol, further preferably 10 to 40 mol, each with respect to the total 100 mol of constitutional units (1), (2), and (3) (i.e., total 100 mol of monomers (1), (2), and (3) used in the polymerization).

While the weight average molecular weight of copolymer (1-2-3) is not particularly limited, it is preferably 10,000 to 500,000, more preferably 10,000 to 100,000, further preferably 10,000 to 50,000. The weight average molecular weight can be determined based on polyethylene glycol by gel permeation chromatography using, for example, EcoSEC system (manufactured by Tosoh Corporation) and the like.

The polymer of the present invention may contain, as long as the effect of the present invention is not impaired, other constitutional units derived from other monomers different from the aforementioned monomer (1) to monomer (3). Only one kind of other monomer may be used, or two or more kinds thereof may be used in combination. Other monomer is not particularly limited, and examples thereof include isobornyl (meth)acrylate, and the like. The amount of other constitutional unit in the polymer of the present invention is preferably not more than 20 mol% with respect to the total constitutional units. More preferably, the polymer of the present invention does not contain other constitutional units.

The polymer of the present invention is
preferably at least one selected from the group consisting of copolymer (1-2a), copolymer (1-2b), and copolymer (1-2-3),
more preferably at least one selected from the group consisting of copolymer (1-2b) and copolymer (1-2-3),
further preferably at least one selected from the group consisting of copolymer (1-2b) and copolymer (1-2a-3).

The explanations for copolymer (1-2a), copolymer (1-2b), and copolymer (1-2-3) (including copolymer (1-2a-3)), and also monomers thereof, are as described above.

The polymer of the present invention can be produced by known methods (e.g., the method described in WO 2018/216628).

The polymer of the present invention contained in a nucleic acid solution can be easily used as a stabilizer.

Methods for containing the polymer of the present invention in a nucleic acid solution include, for example,
(i) a method of adding the polymer of the present invention to an already prepared nucleic acid solution and dissolving the polymer,
(ii) a method of dissolving the polymer of the present invention in advance in a solvent such as a buffer that dissolves a nucleic acid and dissolving the nucleic acid therein,
(iii) a method of placing the polymer of the present invention in advance in a container in which a nucleic acid solution is prepared and then adding the nucleic acid solution thereto to dissolve the polymer,
and the like.

The amount of the stabilizer of the present invention (that is, the polymer of the present invention) is determined by the concentration in the nucleic acid solution described below. The concentration of the stabilizer of the present invention in the aforementioned solution is preferably 0.0001 to 10 w/v%, more preferably 0.001 to 1 w/v%, further preferably 0.01 to 0.09 w/v%, from the viewpoint of the stabilizing effect. When two or more kinds of the stabilizers of the present invention are used, the aforementioned concentration refers to the total of the concentrations of the two or more kinds of the stabilizers. When two or more kinds of the following other components are used, the concentrations described for such other components also refer to the total of the aforementioned concentrations of the two or more kinds of the components.

The nucleic acid to be stabilized with the stabilizer of the present invention may be either RNA or DNA, and is preferably RNA. Examples of the RNA include mRNA, tRNA, rRNA, siRNA, ddRNA, dsRNA, ssRNA, ssRNA-RT, and the like. Here, the nucleic acid may be artificially synthesized by chemical synthesis, in vitro synthesis (e.g., reverse transcription reaction), PCR, or the like, or may be prepared from cells, microorganisms, viruses, or the like by known methods. Here, the cells, microorganisms, viruses, and the like may be those collected from human, animals, or plants in the natural world or environment, or may be those obtained by isolation and culture.

### [Nucleic acid solution]

The present invention further provides a nucleic acid aqueous solution containing the stabilizer of the present invention and a nucleic acid.

The concentration of the stabilizer of the present invention in the nucleic acid solution is 0.0001 to 10 w/v%, more preferably 0.001 to 1 w/v%, further preferably 0.01 to 0.09 w/v%, as described above.

From the viewpoint of stabilizing effect, the concentration of the nucleic acid in the nucleic acid solution is 1 to 10⁸ copies/µL, more preferably 1 to 10⁵ copies/µL, further preferably 1 to 10³ copies/µL.

From the viewpoint of storage stability of nucleic acid, the storage temperature of the nucleic acid solution is preferably -80°C to 40°C, more preferably -20°C to 40°C, further preferably 0°C to 40°C.

The nucleic acid in the nucleic acid solution may be either RNA or DNA, and RNA is preferred. Examples of the RNA include mRNA, tRNA, rRNA, siRNA, ddRNA, dsRNA, ssRNA, ssRNA-RT, and the like. The nucleic acid may be artificially synthesized by, for example, chemical synthesis, in vitro synthesis (e.g., reverse transcription reaction), PCR, or the like, and may be provided as a virus, fungus body, cell, body fluid, tissue, etc., or a suspension of any of these, or a nucleic acid extract, etc., prepared from any of these. The virus, fungus body, cell, body fluid, tissue, and the like may be those collected from human, animals, or plants in the natural world or environment, or may be those obtained by isolation and culture.

The nucleic acid solution may contain other components as long as the effects of the present invention are not impaired. Examples of other component include polyol, polyether, protein, salts, buffer, surfactant, solvent, biochemical reagent, dyes, preservative, oil, solid support, and the like.

Examples of the polyol include glycerol, sucrose, glucose, and the like.

Examples of the polyether include polyoxyethylene glycol and the like.

Examples of the protein include albumin, gelatin, casein, enzyme, and the like.

Examples of the salts include amino acid, peptides, alkali metal salt, alkaline earth metal salt, organic acids such as ethylenediaminetetraacetic acid and the like, and the like.

Examples of the buffer include Tris-HCl buffer, Good's buffer, glycine buffer, borate buffer, TE buffer, TAE buffer, TBE buffer, SSC buffer, and the like.

Examples of the surfactant include polyoxyethylene alkyl ether, sorbitan polyoxyethylene monoalkyl ether, alkylbetaine, and the like.

Examples of the solvent include water and organic solvents. Examples of the organic solvent include ethanol, propanol, isoamyl alcohol, glycerol, dimethylformamide, dimethylsulfoxide, tetrahydrofuran, chloroform, phenol, and the like. The solvent is preferably water. The nucleic acid solution is preferably a nucleic acid aqueous solution, more preferably an RNA aqueous solution. The nucleic acid aqueous solution (particularly RNA aqueous solution) may further contain an organic solvent.

Examples of the biochemical reagent include flavins and the like.

Examples of the dyes include nucleic acid staining reagents such as ethidium bromide, SYBR^{™} Green I and the like, fluorescent dyes such as ROX^{™} Dye, and the like, colorants such as Orange G, bromophenol blue, xylene cyanol FF, and the like, and the like.

Examples of the preservative include sodium azide, parahydroxybenzoic acid preparation, dehydroacetic acid preparation, Proclin preparation, and the like.

Examples of the oil include mineral oil and the like.

Examples of the solid support include silica beads, magnetic beads, and the like.

### [Method for improving storage stability of nucleic acid in nucleic acid solution]

The present invention provides a method for improving the storage stability of a nucleic acid by adding the stabilizer of the present invention to a nucleic acid solution at a concentration of 0.0001 to 10 w/v% (hereinafter sometimes abbreviated as the "improving method of the present invention").

In the improving method of the present invention, the concentration of the stabilizer of the present invention in the nucleic acid solution is 0.0001 to 10 w/v%, preferably 0.001 to 1 w/v%, more preferably 0.01 to 0.09 w/v%, from the viewpoint of stabilizing effect. In the improving method of the present invention, the description of the nucleic acid solution other than the concentration of the stabilizer of the present invention is the same as above.

### [Nucleic acid amplification method]

The present invention provides a nucleic acid amplification method including performing a nucleic acid amplification reaction using a solution containing the stabilizer of the present invention and a nucleic acid. The aforementioned nucleic acid is preferably RNA.

The nucleic acid amplification method is preferably a polymerase chain reaction method (PCR method), more preferably a reverse transcription polymerase chain reaction method in which the amplification target is RNA (RT-PCR method). The RT-PCR method may also be a reverse transcription-quantitative PCR method (RT-qPCR method).

The PCR method (particularly RT-PCR method) is well known to those of ordinary skill in the art. No particular limitation is imposed on the conditions and reagents (e.g., buffer, substrate, primer, reverse transcriptase, DNA polymerase, fluorescent DNA staining reagent, fluorescent probe, passive reference, etc.) used in PCR, and those of ordinary skill in the art can perform PCR using a nucleic acid solution and known reagents for PCR.

### [Example]

The present invention is explained in more detail in the following by referring to Examples and the like, which are not to be construed as limitative.

### [Synthetic Example 1]

2-Methacryloyloxyethylphosphorylcholine (hereinafter referred to as "MPC") (8.4 g) as monomer (1), butyl methacrylate (hereinafter referred to as "BMA") (2.1 g) as monomer (2), and glycerol monomethacrylate (hereinafter referred to as "GLM") (4.5 g) as monomer (3) (monomer (1)/monomer (2)/monomer (3) = 40/40/20 (molar ratio)) were weighed into a glass flask for polymerization, purified water (42.5 g) and ethanol (42.5 g) were added to dissolve monomers (1) to (3), and Perroyl SA (manufactured by NOF CORPORATION, hereinafter referred to as "PRSA") (0.15 g) was added to the obtained solution. After the inside of the reaction container was sufficiently replaced with nitrogen, polymerization was performed by heating at 70°C for 7 hr while stirring. The obtained reaction solution was ice-cooled and added dropwise to acetone to precipitate a polymer. The precipitate was collected by filtration, washed with acetone, and vacuum dried to give a homopolymer (hereinafter referred to as "polymer 1") as a white powder. The weight average molecular weight of polymer 1 was 21,000 based on polyethylene glycol as measured by gel filtration chromatography (hereinafter referred to as "GPC") under the below-mentioned conditions.

### [Synthetic Example 2]

MPC (6.0 g) as monomer (1) and methacrylic acid (hereinafter referred to as "MAc") (4.0 g) as monomer (2a) (monomer (1)/monomer (2) = 30/70 (molar ratio)) were weighed into a glass flask for polymerization, purified water (90.0 g) was added to dissolve monomers (1) and (2), and PRSA (0.78 g) was added to the obtained solution. Thereafter, in the same manner as in Synthetic Example 1, a copolymer (hereinafter referred to as "polymer 2") was obtained. The weight average molecular weight of polymer 2 was 680,000 based on polyethylene glycol as measured by GPC under the below-mentioned conditions.

### [Synthetic Example 3]

MPC (25.3 g) as monomer (1) and methoxypolyethylene glycol methacrylate (BLEMMER PME, manufactured by NOF CORPORATION, number average molecular weight: about 500, n in the formula (2): about 9, hereinafter referred to as "PEGMA") (4.7 g) as monomer (2) (monomer (1)/monomer (2) = 90/10 (molar ratio)) were weighed into a glass flask for polymerization, purified water (70.0 g) was added to dissolve monomers (1) and (2), and PRSA (0.23 g) was added to the obtained solution. Thereafter, in the same manner as in Synthetic Example 1, a copolymer (hereinafter referred to as "polymer 3") was obtained. The weight average molecular weight of polymer 3 was 135,000 based on polyethylene glycol as measured by GPC under the below-mentioned conditions.

### [Synthetic Example 4]

MPC (6.0 g) as monomer (1) and stearyl methacrylate (hereinafter referred to as "SMA") (3.3 g) as monomer (2) (monomer (1)/monomer (2) = 80/20 (molar ratio)) were weighed into a glass flask for polymerization, ethanol (85.0 g) was added to dissolve monomers (1) and (2), and azobis(isobutyronitrile) (0.06 g) was added as a polymerization initiator to the obtained solution. Thereafter, in the same manner as in Synthetic Example 1, a copolymer (hereinafter referred to as "polymer 4") was obtained. The weight average molecular weight of polymer 4 was 43,000 based on polyethylene glycol as measured by GPC under the below-mentioned conditions.

### <GPC measurement>

GPC measurement of the polymers 1 to 4 obtained in Synthetic Examples 1 to 4 was performed under the following conditions.
GPC system: EcoSEC system (manufactured by Tosoh Corporation)
column: Shodex OHpak SB-802.5HQ (manufactured by Showa Denko K.K.), and SB-806HQ (manufactured by Showa Denko K.K.) connected in tandem
eluent: 20 mM sodium phosphate buffer (pH 7.4)
detector: differential refractive index detector
molecular weight standard: EasiVial PEG/PEO (manufactured by Agilent Technologies)
flow rate: 0.5 mL/min
column temperature: 40°C
sample: obtained polymer diluted with eluent to final concentration of 0.1 wt%
injection volume: 100 µL

The monomers used in Synthetic Examples 1 to 4 and molar ratios thereof, and weight average molecular weights of the obtained polymers are summarized in Table 1.

**[Table 1]**

| | monomer (1) | monomer (2) | monomer (3) | molar ratio of monomers | weight average molecular weight |
|---|---|---|---|---|---|
| polymer 1 | MPC | BMA | GLM | 40/40/20 | 21,000 |
| polymer 2 | MPC | MAc | - | 30/70/0 | 680,000 |
| polymer 3 | MPC | PEGMA | - | 90/10/0 | 135,000 |
| polymer 4 | MPC | SMA | - | 80/20/0 | 43,000 |

| | | | | | |
|---|---|---|---|---|---|
| molar ratio of monomers = monomer (1)/monomer (2) /monomer (3) MPC: 2-methacryloyloxyethyl phosphorylcholine BMA: butyl methacrylate GLM: glycerol monomethacrylate MAc: methacrylic acid PEGMA: methoxypolyethylene glycol methacrylate SMA: stearyl methacrylate | | | | | |

### [Examples 1 to 4]

Examples are shown in which the nucleic acid is RNA and the nucleic acid amplification method is RT-qPCR.

<Preparation of nucleic acid solution>

The nucleic acid solution shown in Table 2 was prepared. This nucleic acid solution was prepared while cooling on ice. Solutions of RNA and polymer were used to prepare nucleic acid solutions. Table 2 shows the amounts of RNA solution and polymer solution added, and the final concentrations of RNA and polymer.

**[Table 2]**

| composition | PW*¹ | | 89 µL | | | |
|---|---|---|---|---|---|---|
| | RNA*² | | 10 µL (final concentration 10 copies/uL) | | | |
| | polymer | kind | polymer 1 | polymer 2 | polymer 3 | polymer 4 |
| | | amount added | 1.0 µL (final concentration 0.05 w/v%) | | | |
| total | | | 100 µL | | | |

| | | | | | | |
|---|---|---|---|---|---|---|
| *1 Distilled Water, Nuclease-free (manufactured by NIPPON GENE CO., LTD.) *2 2019-nCov one-step RT-qPCR N2 set positive control RNA (manufactured by NIHON GENE RESEARCH LABORATORIES Inc.) | | | | | | |

### <Storage of Nucleic Acid Solution>

The prepared nucleic acid solution was stored at 4°C for 4 days or at 37°C for 11 days.

### <Preparation of composition for nucleic acid amplification>

A composition for nucleic acid amplification was prepared by mixing the components shown in Table 3. This composition for nucleic acid amplification was prepared while cooling on ice.

**[Table 3]**

| reagent name | amount added*¹ |
|---|---|
| Distilled Water, Nuclease-free (manufactured by NIPPON GENE CO., LTD.) | 3.35 µL |
| 5x Buffer for rTth/TTx(DNA/RNA) (manufactured by TOYOBO CO., LTD.) | 4.00 µL |
| 2 mM dNTPs (manufactured by TOYOBO CO., LTD.) | 4.00 µL |
| 50 mM Mn(OAc)₂ (manufactured by TOYOBO CO., LTD.) | 1.00 µL |
| first primer (manufactured by FUJIFILM Wako Pure Chemical Corporation) | 0.60 µL |
| second primer (manufactured by FUJIFILM Wako Pure Chemical Corporation) | 0.60 µL |
| TaqMan Probe (manufactured by FUJIFILM Wako Pure Chemical Corporation) | 0.80 µL |
| Hot Start TTx DNA Polymerase (manufactured by TOYOBO CO., LTD.) | 0.25 µL |
| 50x ROX (manufactured by NIPPON GENE CO., LTD.) | 0.40 µL |
| total | 15.0 µL |

| | |
|---|---|
| *1 amount added: amount added per well | |

### <Preparation of RT-qPCR reaction mixture>

5 µL per well of a nucleic acid solution before storage (i.e., immediately after production), a nucleic acid solution stored at 4°C for 4 days, or a nucleic acid solution stored at 37°C for 11 days was added to the prepared composition for nucleic acid amplification to prepare RT-qPCR reaction mixtures.

### <RT-qPCR>

RT-qPCR was performed using the RT-qPCR reaction mixtures obtained as described above and a qPCR device ("StepOnePlus^{™} Real-Time PCR System" manufactured by Applied Biosystems), and the Ct value of the RT-qPCR reaction mixtures was measured. Here, "Ct value" is the number of cycles required for the detected fluorescent signal to exceed the threshold (background). A lower Ct value indicates a higher amount of RNA. The measurements were performed three times for each RT-qPCR reaction mixture. The results are shown in the following Tables 5 and 6. The temperature program is as shown in the following Table 4.

**[Table 4]**

| step | reaction conditions | |
|---|---|---|
| 1 | 90°C, 30 sec | |
| 2 | 60°C, 5 min | |
| 3 | 95°C, 1 min | |
| 4 | 95°C, 15 sec | 60 cycles |
| 5 | 60°C, 30 sec | |

### [Comparative Example 1]

In Comparative Example 1, the same operations as in Examples 1 to 4 were performed except that 1.0 µL of Distilled Water, Nuclease-free, was used instead of 1.0 µL of the polymer solution. The results are shown in the following Tables 5 and 6.

**[Table 5]**

| stored at 4°C | | Ct value (n = 3) | |
|---|---|---|---|
| | kind of polymer | nucleic acid solution before storage | nucleic acid solution after 4 days of storage |
| Example 1 | polymer 1 | 33.4±0.23 | 33.3±0.37 |
| Example 2 | polymer 2 | 34.1±0.18 | 34. 6±0.18 |
| Example 3 | polymer 3 | 32.7±0.16 | 32.5±0.25 |
| Example 4 | polymer 4 | 32.8±0.90 | 35.7±1.10 |
| Comparative Example 1 | none | 33.8±0.45 | -*¹ |

| | | | |
|---|---|---|---|
| *1 Ct value was not obtained | | | |

**[Table 6]**

| stored at 37°C | | Ct value (n = 3) | |
|---|---|---|---|
| | kind of polymer | nucleic acid solution before storage | nucleic acid solution after 11 days of storage |
| Example 1 | polymer 1 | 33.3±0.16 | 37.5±0.93 |
| Example 2 | polymer 2 | 33.8±0.59 | 38.8*¹ |
| Example 3 | polymer 3 | 32.7±0.16 | 37.7±0.60 |
| Example 4 | polymer 4 | 32.7±0.50 | 36.7*¹ |
| Comparative Example 1 | none | 33.8±0.30 | -*² |

| | | | |
|---|---|---|---|
| *1 Ct value was obtained in only one RT-qPCR reaction mixture *2 Ct value was not obtained | | | |

As shown in Tables 5 and 6, no Ct value was obtained after storage of the nucleic acid solution in Comparative Example 1, in which the stabilizers of the present invention (polymers 1 to 4) were not used, whereas Ct values were obtained even after storage of the nucleic acid solution in Examples 1 to 4, in which the stabilizers of the present invention (polymers 1 to 4) were used. In particular, in Examples 1 and 3, Ct values were obtained for all three RT-qPCR reaction mixtures, after storage at both 4°C and 37°C. Furthermore, as shown in Table 5, almost no change was found in Ct values before and after storage at 4°C in Examples 1 to 4. These results demonstrate that the stabilizer of the present invention stabilizes a nucleic acid simply by adding same to a solution of the nucleic acid.

### [Industrial Applicability]

The stabilizer of the present invention is useful for improving the storage stability of nucleic acids in solutions.

This application is based on a patent application No. 2023-166492 filed in Japan, the contents of which are encompassed in full herein.

## Claims

1. A stabilizer for a nucleic acid solution, which is a polymer comprising a constitutional unit derived from a monomer represented by the formula (1): 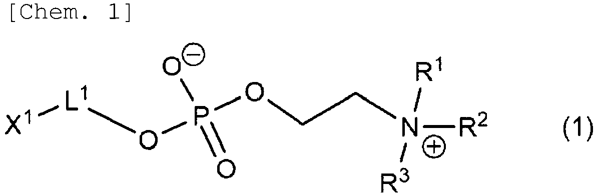 wherein
X¹ is a (meth)acryloyloxy group or a (meth)acryloylamino group,
L¹ is an alkylene group having 2 to 4 carbon atoms and optionally having one hydroxy group, or an alkyleneoxyalkylene group having 2 to 4 carbon atoms, and
R¹ to R³ are each independently an alkyl group having 1 to 3 carbon atoms.

2. The stabilizer according to claim 1, wherein the polymer is a copolymer further comprising a constitutional unit derived from a monomer represented by the formula (2): 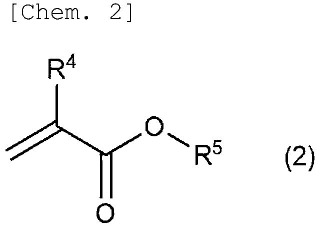 wherein
R⁴ is a hydrogen atom or a methyl group, and
R⁵ is is a hydrogen atom, an alkyl group having 1 to 20 carbon atoms, or *-(OCH₂CH₂)ₙOR⁶ group (in the formula, * is a binding position, n is a number from 1 to 10, and R⁶ is a hydrogen atom, a methyl group, or an ethyl group.

3. The stabilizer according to claim 2, wherein the polymer is a copolymer further comprising a constitutional unit derived from a monomer represented by the formula (3): 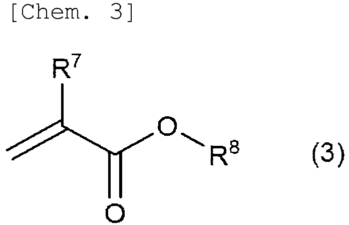 wherein
R⁷ is a hydrogen atom or a methyl group, and
R⁸ is an alkyl group having 3 to 6 carbon atoms, and two or more hydroxy groups.

4. The stabilizer according to claim 1, wherein the nucleic acid solution is an RNA solution.

5. A method for improving the storage stability of a nucleic acid, comprising adding the stabilizer according to any one of claims 1 to 4 to a solution of the nucleic acid at a concentration of 0.0001 to 10 w/v%.
